(19) Europäisches Patentamt
European Patent Office
Office européen des brevets

(11) Veröffentlichungsnummer: **0 249 024**
**B1**

(12) # EUROPÄISCHE PATENTSCHRIFT

(45) Veröffentlichungstag der Patentschrift: **06.12.89**

(51) Int. Cl.⁴: **C 07 C 147/06,** C 07 C 147/103, C 08 G 75/20

(21) Anmeldenummer: **87106378.0**

(22) Anmeldetag: **30.04.87**

(54) **Aromatische, Keton- oder Aldehydgruppen enthaltende (Poly)Sulfone und Verfahren zu ihrer Herstellung.**

(30) Priorität: **13.05.86 DE 3616065**

(43) Veröffentlichungstag der Anmeldung: **16.12.87 Patentblatt 87/51**

(45) Bekanntmachung des Hinweises auf die Patenterteilung: **06.12.89 Patentblatt 89/49**

(84) Benannte Vertragsstaaten: **DE FR GB IT NL**

(56) Entgegenhaltungen: **DE-A-2 127 660**

**CHEMICAL ABSTRACTS, Band 73, Nr. 13, 28 September 1970, Columbus, Ohio, USA S.S. GITIS et al. "Synthesis of isomeric bis(aminophenylsulfonyl) Benzophenones" Seite 310, Spalte 2, Seite 311, Spalte 1, Zusammenfassung-Nr. 66 196g**

(73) Patentinhaber: **BAYER AG, D-5090 Leverkusen 1 Bayerwerk (DE)**

(72) Erfinder: **Reuter, Knud, Dr., Scheiblerstrasse 99, D-4150 Krefeld (DE)**

**EP 0 249 024 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung betrifft ein Verfahren zur Herstellung aromatischer, Keton- oder Aldehydgruppen enthaltender (Poly)Sulfone sowie neue aromatische (Poly)Sulfone.

Die Herstellung aromatischer Sulfone ist bekannt (z. B. Ullmann's Encyclopädie der Technischen Chemie, 4. Auflage, Bd. 22, S. 323), z. B. Oxidation von Sulfiden bzw. Sulfoxiden, Sulfonierung aromatischer Kohlenwasserstoffe, Umlagerung von Sulfonsäureestern zu Hydroxydiphenylsulfonen, die Addition von Sulfinsäuren an Chinone und Chinonimine sowie die nucleophile Substitution von Chlor in o- oder p-Chlornitrobenzolen.

Die Herstellung von Sulfonen aus Nitroaromaten durch Substitution einer $NO_2$-Gruppe ist in einigen speziellen Fällen ebenfalls bekannt, z. B. J. Chem. Soc. *1936*, 216, J. Chem. Soc. 1937, 246, J. Chem. Soc. *1939*, 902.

Bekanntlich reicht eine zweite Nitrogruppe in o- oder p- Stellung aus, um den Austausch einer $NO_2$-Gruppe gegen Sulfinat zu ermöglichen (J. Org. Cham. *41*, (9), 1560 (1976), C.A. *87*, 565 (1977).

Es wurden nun neue, Ketogruppen enthaltende aromatische Sulfone und ein Verfahren zur Herstellung von Keton- oder Aldehydgruppen enthaltenden (Poly)Sulfonen durch nucleophile aromatische Substitution von Nitrogruppen gefunden.

Gegenstand der Erfindung ist somit ein Verfahren zur Herstellung von Keton- bzw. Aldehydgruppen enthaltenden (Poly)Arylsulfonen, das dadurch gekennzeichnet ist, daß Arylsulfinsäuren oder ihre Salze mit aromatischen Nitroverbindungen der Formel (I)

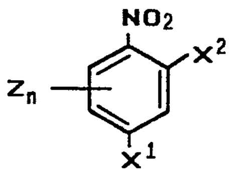

(I),

in welcher

| | |
|---|---|
| $X^1$ | H oder -CO-$R^1$ und |
| $X^2$ | H oder -CO-$R^2$ ist und |
| $X^1$ und $X^2$ | nicht gleichzeitig H sein dürfen und |
| $R^1$ | für H oder einen gegebenenfalls mit $C_1$-$C_4$-Alkyl, F, Cl, Br, J oder $NO_2$ substituierten $C_6$-$C_{14}$-Arylrest, |
| $R^2$ | für einen gegebenenfalls mit $C_1$-$C_4$-Alkyl, F, Cl, Br, J oder $NO_2$ substituierten $C_6$-$C_{14}$-Arylrest, |
| Z | für $C_1$-$C_4$-Alkyl, $C_6$-$C_{10}$-Aryl, $C_1$-$C_4$-Alkoxy, $C_6$-$C_{10}$-Aryloxy), $C_1$-$C_4$-Dialkylamino, $C_6$-$C_{10}$-Aryl-$C_1$-$C_4$-alkylamino, $C_6$-$C_{10}$-Diarylamino und |
| n | für die Zahl 0, 1, 2, 3 oder 4 steht, |

umgesetzt werden.

Erfindungsgemäß können Arylsulfinsäuren oder deren Salze der Formel (II)

$Ar^1\ [SO_2M]_m$ (II),

in welcher

| | |
|---|---|
| $Ar^1$ | für gegebenenfalls mit $C_1$-$C_4$-Alkyl, oder Halogen wie Cl, Br oder $NO_2$ substituiertes $C_6$-$C_{14}$-Aryl (m = 1), oder $C_6$-$C_{14}$-Arylen (m = 2) steht, |
| m | für die Zahl 1 oder 2 steht und |
| M | für Wasserstoff oder Alkali wie Li, Na, K und die $NR_4$-Gruppe steht, in welcher R für $C_1$-$C_4$-Alkyl steht, |

eingesetzt werden.

Beispiele für erfindungsgemäß einzusetzende Arylsulfinsäuren sind:

Benzolsulfinsäure, p-Toluolsulfinsäure, p-Chlorbenzolsulfinsäure, p-Nitrobenzolsulfinsäure, Benzol-1,3-disulfinsäure, Toluol-2,4-disulfinsäure, Diphenylether-4,4'-disulfinsäure, Diphenylsulfon-3,3'-disulfinsäure usw.

Ein weiterer Gegenstand der Erfindung ist ein Verfahren zur Herstellung von aromatischen (Poly)Sulfonen, das dadurch gekennzeichnet ist, daß Arylsulfinsäuren der Formel (II) oder ihre Salze mit Nitrobenzophenonen der Formel (III)

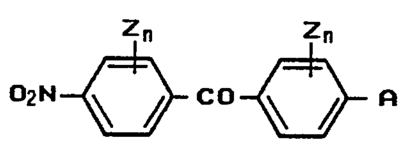

(III),

in welcher

Z und n die bei Formel (I) genannte Bedeutung haben und
A für Wasserstoff, Halogen wie F, Cl, Br, J oder eine Nitrogruppe steht,

wobei für den Fall, daß A für Halogen oder eine Nitrogruppe steht, die Gruppe A ebenfalls mit der Arylsulfinsäure umgesetzt werden kann,
umgesetzt werden.

Ein weiteres erfindungsgemäßes Verfahren zur Herstellung von aromatischen (Poly)Sulfonen mit Keton- oder Aldehydgruppen ist dadurch gekennzeichnet, daß mit den oben genannten Nitroverbindungen der Formel (I) aromatische Disulfinsäuren (bzw. deren Salze) der Formel (IV) und (V)

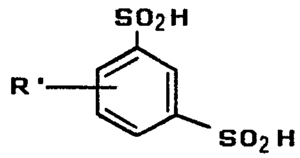

(IV),

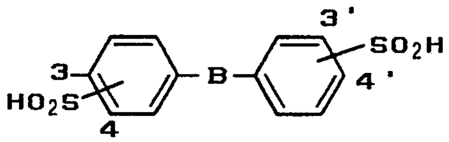

(V),

in welcher

R' = H, $C_{1-18}$-Alkyl,
B = O oder $SO_2$ ist und
$X_1$, $X_2$, und n die bei Formel (I) angegebene Bedeutung haben,

umgesetzt werden.

Bevorzugt werden in allen bisher genannten erfindungsgemäßen Verfahren zur Herstellung von aromatischen (Poly)Sulfonen die Lithium-, Natrium-, Kalium- oder Ammoniumsalze der Sulfinsäuren oder deren Salze mit organischen Basen, also solche mit $NR"_4$-Kationen, R"=$C_1$-$C_4$-Alkyl oder $C_7$-$C_{12}$-Aralkyl, wobei die Reste R" gleich oder verschieden sein können, wie z. B. in Benzyltrimethylammonium.

Besonders bevorzugt ist es bei allen bisher geschilderten erfindungsgemäßen Verfahren, die Reaktion in einem dipolaren aprotischen Lösungsmittel, wie Dimethylsulfoxid (DMSO) oder Dimethylformamid, ganz besonders bevorzugt in DMSO, durchzuführen, oder in Lösungsmittelgemischen, die überwiegend aus dipolaren aprotischen Lösungsmitteln, bevorzugt DMSO, bestehen, zu arbeiten.

Gegenstand der Erfindung sind weiterhin aromatische Sulfon-Ketone der Formel (VI)

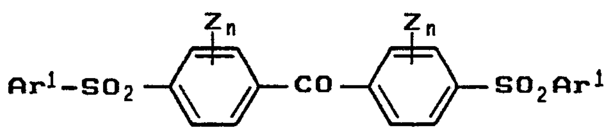

(VI),

in der

$Z_n$ die bei Formel I angegebene Bedeutung hat und
$Ar^1$ ein monofunktioneller aromatischer $C_6$-$C_{14}$-Rest, jedoch nicht Phenyl, 3- oder 4-Nitrophenyl oder 3- oder 4-Aminophenyl, ist.

Gegenstand der Erfindung sind schließlich Polysulfon-Ketone der Formel (VII)

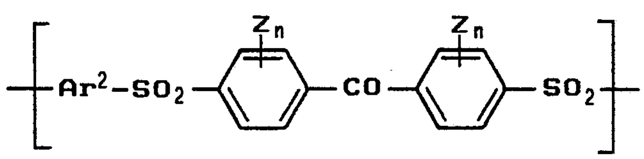

(VII),

in welcher

$Z_n$ die bei Formel (I) angegebene Bedeutung hat und
$Ar^2$ einen difunktionellen aromatischen $C_6$-$C_{14}$-Rest darstellt.

Eine Auswahl typischer Reaktionspartner sind beispielsweise 4,4'-Dinitrobenzophenon, 4-Nitro-4'-chlorbenzophenon, 4-Nitro-4'-fluorbenzophenon, 4'-Nitrobenzophenon usw.

Die nach dem erfindungsgemäßen Verfahren hergestellten Produkte eignen sich z. B. als Rohstoffe für Kunststoffe, beispielsweise derart, daß die hochmolekularen Produkte zu thermoplastischen Werkstoffen verarbeitet werden, oder derart, daß niedermolekulare Produkte, welche geeignete funktionelle Gruppen tragen (z. B. Cl, $NO_2$, $NH_2$, Carboxyl usw.) mit dazu passenden Reaktionspartnern zu hochmolekularen Produkten umgesetzt und diese anschließend zu thermoplastischen Werkstoffen verarbeitet werden.

## Beispiele

### Beispiel 1

21,94 g Natriumbenzolsulfinat (70,2 %-ig) werden in 120 ml DMSO und 80 ml Toluol azeotrop am Wasserabscheider entwässert. Für die Entfernung restlicher Spuren Wasser wird anschließend ein Soxhlet-Extraktor, der mit Molekularsieb 4 Å gefüllt ist, aufgesetzt und 1 h darunter zum Rückfluß erhitzt. Danach wird eine Destillationsapparatur aufgesetzt und bis zu einer Innentemperatur von 145°C die Hauptmenge Toluol und wenig DMSO abdestilliert. Anschließend werden 26,2 g 4-Chlor-4'-nitro-benzophenon (Molverh. 1 : 1) zugegeben und das Gemisch 24 h auf ca. 150°C erhitzt. Danach wird das Reaktionsgemisch in Methanol eingegossen; man erhält 11,2 g einer Ausfällung, die nach Elementaranalyse ein Gemisch aus den beiden folgenden Verbindungen darstellt:

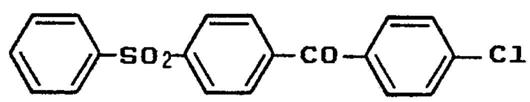

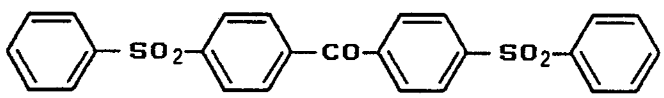

### Beispiel 2

21,9 g Natrium-p-toluolsulfinat (76,6 %-ig) werden entsprechend dem Verfahren in Bsp. 1 in 120 ml DMSO/80 ml Toluol entwässert. Nach Eindestillieren bis 145°C werden 26,2 g 4-Chlor-4'-nitrobenzophenon (Molverh. 1 : 1) zugegegeben und das Gemisch 24 h auf 150°C erhitzt. Nach Abkühlen wurde filtriert und die Mutterlauge in Methanol eingegossen. Man erhält 5,8 g eines Gemisches, das nach NMR-Spektrum und Elementaranalyse aus folgenden Verbindungen im Molverhältnis von etwa 2 : 1 besteht:

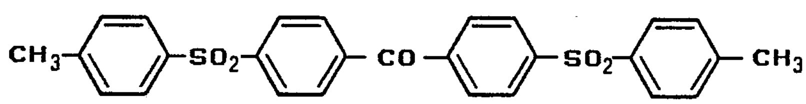

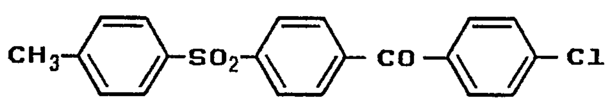

### Beispiel 3

15,7 g 4-Chlor-4'-nitrobenzophenon und 39,4 g Natrium-p-toluolsulfinat (Molverh. 1 : 3) werden in 100 ml trockenem DMSO 24 h auf 150°C erhitzt. Der nach Abkühlen auf Raumtemperatur entstehende Niederschlag wurde 2 x mit Methanol gewaschen, anschließend 1 h mit Wasser aufgekocht, heiß filtriert und nochmals mit Wasser gewaschen. Man erhält 11,8 g eines Rohproduktes, das aus Chlorbenzol umkristallisiert werden kann, Schmp. nach Umkristallisation: 274 - 277°C. Nach IR- und NMR-Spektrum sowie Elementaranalyse wurde folgende Verbindung erhalten:

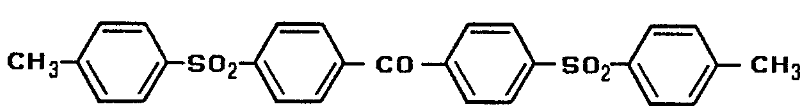

**Beispiel 4**

35 g Natrium-toluolsulfinat (76,6 %-ig) werden entsprechend dem in Beispiel 1 angegebenen Verfahren in 120 ml Toluol/200 ml DMSO entwässert. Anschließend wurden 24,2 g 4-Nitrobenzaldehyd zugegeben und das Reaktionsgemisch 8 h auf 150°C erhitzt. Danach wird mit Wasser verdünnt, mit Methylenchlorid extrahiert und die organische Phase eindestilliert. Der Rückstand (ca. 4 g) wird mit Wasser und Diethylether gewaschen und anhand des Schmelzpunktes (150°C), des Massenspektrums und der Elementaranalyse als folgende Verbindung identifiziert.

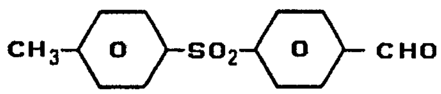

Die Reinheit beträgt etwa 80 %.

**Beispiel 5**

21,9 g Natrium-p-toluolsulfinat (76,6 %-ig) werden entsprechend dem Verfahren in Beispiel 1 in 240 ml DMSO und 200 ml Toluol entwässert. Anschließend werden 12,25 g 4-Fluor-4'-nitrobenzophenon zugegeben und das Reaktionsgemisch 8 Stunden auf ≈ 150°C erhitzt. Nach Abkühlen wurde in Methanol gefällt und der Niederschlag mit Wasser und Methanol gewaschen. Es wurden 6 g Produkt erhalten, welches identisch mit dem aus Beispiel 3 war (NMR, IR, Elementaranalyse, Schmelzpunkt).

**Patentansprüche**

1. Verfahren zur Herstellung von Keton- oder Aldehydgruppen enthaltenden (Poly)Arylsulfonen, dadurch gekennzeichnet, daß Arylsulfinsäuren oder ihre Salze mit aromatischen Nitroverbindungen dar Formel (I)

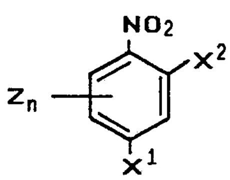

(I),

in welcher

$X^1$ H oder -CO-$R^1$ und
$X^2$ H oder -CO-$R^2$ ist, und
$X^1$ und $X^2$ nicht gleichzeitig H sein dürfen und
$R^1$ für H oder einen gegebenenfalls mit $C_1$-$C_4$-Alkyl, F, Cl, Br, J oder $NO_2$ substituierten $C_6$-$C_{14}$-Arylrest,
$R^2$ für einen gegebenenfalls mit $C_1$-$C_4$-Alkyl, F, Cl, Br, J oder $NO_2$ substituierten $C_6$-$C_{14}$-Arylest,

Z für $C_1$-$C_4$-Alkyl, $C_6$-$C_{10}$-Aryl, $C_1$-$C_4$-Alkoxy, $C_6$-$C_{10}$-Aryloxy, $C_1$-$C_4$-Dialkylamino, $C_6$-$C_{10}$-Aryl-$C_1$-$C_4$-alkylamino, $C_6$-$C_{10}$-diarylamino und
n für eine ganze Zahl von 0 bis 4 steht,

umgesetzt werden.

2. Verfahren zur Herstellung von aromatischen (Poly)Sulfonketonen, dadurch gekennzeichnet, daß Arylsulfinsäuren oder ihre Salze mit Nitrobenzobenzophenonen der Formel (III)

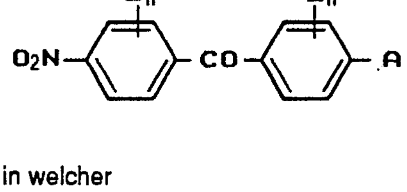

(III),

in welcher

$Z_n$ die in Anspruch 1 genannte Bedeutung hat und

A ein Wasserstoffatom, ein Halogen oder eine Nitrogruppe ist, wobei für den Fall, daß A, F, Cl, Br, J oder eine Nitrogruppe ist, A ebenfalls mit der Arylsulfinsäure umgesetzt werden kann,

umgesetzt werden.

3. Verfahren zur Herstellung von (Poly)aromatischen Keton- oder Aldehydgruppen enthaltenden Sulfonen, dadurch gekennzeichnet, daß die Nitroverbindungen der Formel (I) mit aromatischen Disulfinsäuren (bzw. deren Salzen) der allgemeinen Formeln (IV) und (V)

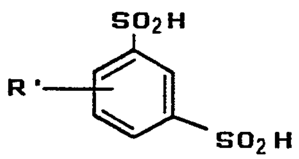

(IV),

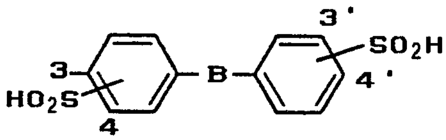

(V),

in welcher

R' H, $C_{1-18}$-Alkyl, Aryl,
B O oder $SO_2$ ist und
X1, $X_2$, Z und ndie bei Formel (I) angegebene Bedeutung haben,

umgesetzt werden.

4. Verfahren zur Herstellung von aromatischen, Keton- oder Aldehydgruppen enthaltenden Sulfonen, dadurch gekennzeichnet, daß Nitroverbindungen der Formel (I) mit Sulfinsäuren (bzw. deren Salzen) der Formel (II)

$Ar^1$ $[SO_2\ M]_{m}1$ (II),

in welcher

$Ar^1$ für gegebenenfalls mit $C_1$-$C_4$-Alkyl, oder Halogen wie Cl, Br oder $NO_2$ substituiertes $C_6$-$C_{14}$-Aryl (m = 1), oder $C_6$-$C_{14}$-Arylen (m = 2) steht,
m für die Zahl 1 oder 2 steht und
M für Wasserstoff oder Alkali wie Li, Na, K und die $NR_4$-Gruppe steht, in welcher R für $C_1$-$C_4$-Alkyl steht.

umgesetzt werden.

5. Verfahren zur Herstellung von aromatischen (Poly)Sulfonen gemäß Anspruch 1, dadurch gekennzeichnet, daß die Lithium-, Natrium-, Kalium- oder Ammoniumsalze der Sulfinsäuren oder deren Salze mit organischen Basen eingesetzt werden.

6. Verfahren gemäß Anspruch 1, dadurch gekennzeichnet, daß die Reaktion in einem dipolaren aprotischen Lösungsmittel, wie Dimethylsulfoxid (DMSO) oder Dimethylformamid durchgeführt wird oder in Lösungsmittelgemischen, die überwiegend aus dipolaren aprotischen Lösungsmitteln bestehen, gearbeitet wird.

7. Aromatische Sulfon-Ketone der Formel (VI)

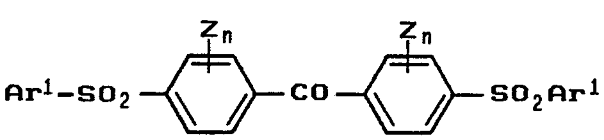

(VI),

in der

$Z_n$ die bei Formel (I) angegebene Bedeutung hat und
$Ar^1$ ein monofunktioneller aromatischer $C_6$-$C_{14}$-Rest, jedoch nicht Phenyl, 3- oder 4-Nitrophenyl oder 3- oder 4-Aminophenyl, ist.

8. Polysulfon-Ketone der Formel (VII)

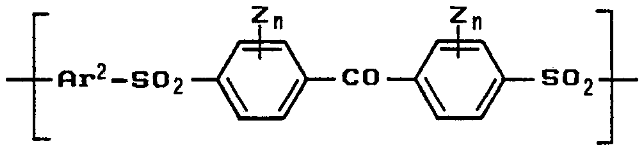

(VII),

in welcher

$Z_n$ die bei Formel (I) angegebene Bedeutung hat und
$Ar^2$ einen difunktionellen aromatischen $C_6$-$C_{10}$-Rest darstellt.

## Claims

1. Process for the preparation of (poly)aryl sulphones containing ketone or aldehye groups, characterised in that aryl sulphinic acids or their salts are reacted with aromatic nitro compounds corresponding to formula (I):

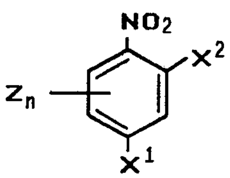

(I),

wherein

$X^1$ denotes H or -CO-$R^1$ and
$X^2$ denotes H or -CO-$R^2$ and
$X^1$ and $X^2$ must not both be H and
$R^1$ denotes H or a $C_6$-$C_{14}$ aryl group optionally substituted with $C_1$-$C_4$ alkyl, F, Cl, Br, I or $NO_2$,
$R^2$ denotes a $C_6$-$C_{14}$ aryl group optionally substituted with $C_1$-$C_4$ alkyl, F, Cl, Br, I or $NO_2$,
Z denotes $C_1$-$C_4$ alkyl, $C_6$-$C_{10}$ aryl, $C_1$-$C_4$ alkoxy, $C_6$-$C_{10}$ aryloxy, $C_1$-$C_4$ dialkylamino, $C_6$-$C_{10}$aryl-$C_1$-$C_4$-alkylamino, or $C_6$-$C_{10}$ diarylamino and
n stands for an integer from 0 to 4.

2. Process for the preparation of aromatic (poly)sulphone ketones, characterized in that arylsulphinic acids or their salts are reacted with nitrobenzobenzophenones corresponding to formula (III):

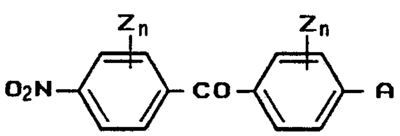

(III),

wherein

$Z_n$ has the meaning indicated in claim 1 and
A denotes a hydrogen atom, a halogen or a nitro group and when A stands for F, Cl, Br, I or a nitro group then A may also be reacted with the arylsulphinic acid.

3. Process for the preparation of (poly)aromatic sulphones containing ketone or aldehyde groups; characterised in that the nitro compounds of formula (I) are reacted with aromatic disulphinic acids (or their salts) corresponding to the general formulae (IV) and (V)

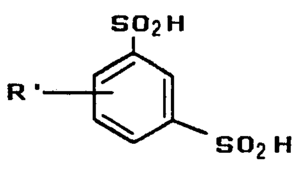

(IV),

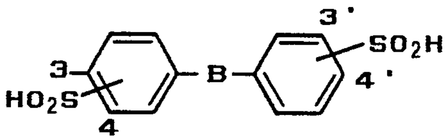 (V),

wherein

R' H, $C_{1-18}$ alkyl or aryl,
B O or $SO_2$ and
$X^1$, $X^2$, Z and n have the meanings indicated for formula (I).

4. Process for the preparation of aromatic sulphones containing ketone or aldehyde groups, characterised in that nitro compounds corresponding to formula (I) are reacted with sulphinic acids (or their salts) corresponding to formula (II):

$Ar^1$ $[SO_2 M]_m$ (II),

wherein
$Ar^1$ stands for $C_6$-$C_{14}$ aryl optionally substituted with $C_1$-$C_4$ alkyl or with halogen such as Cl or Br or with $NO_2$ (when m = 1) or it stands for $C_6$-$C_{14}$ arylene (when m = 2),
m stands for the number 1 or 2 and
M stands for hydrogen or an alkali metal such as Li, Na or K or the group $NR_4$ in which R stands for $C_1$-$C_4$ alkyl.

5. Process for the preparation of aromatic (poly)sulphones according to claim 1, characterised in that the lithium, sodium, potassium or ammonium salts of the sulphinic acids or their salts with organic bases are used.

6. Process according to claim 1, characterised in that the reaction is carried out in a dipolar aprotic solvent such as dimethyl sulphoxide (DMSO) or dimethyl formamide or in solvent mixtures consisting predominantly of dipolar aprotic solvents.

7. Aromatic sulphone ketones corresponding to formula (VI):

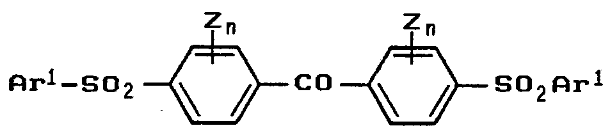 (VI),

wherein

$Z_n$ has the meaning given for formula (I) and
$Ar^1$ denotes a monofunctional aromatic $C_6$-$C_{14}$ group but not phenyl, 3- or 4-nitrophenyl or 3- or 4-aminophenyl.

8. Polysulphone-ketones corresponding to formula (VII):

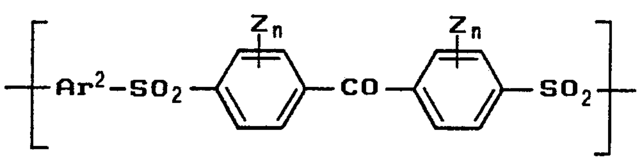 (VII),

wherein

$Z_n$ has the meaning given for formula (I) and
$Ar^2$ denotes a difunctional aromatic $C_6C_{14}$ group.

## Revendications

1. Procédé pour préparer des (poly)arylsulfones contenant des groupes cétones ou aldéhydes, caractérisé en ce qu'on fait réagir des acides arylsulfiniques, ou leurs sels, avec des composés nitrés aromatiques de formule (I):

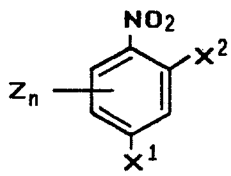

(I),

dans laquelle

| | |
|---|---|
| $X^1$ | représente H ou $-CO-R^1$, et |
| $X^2$ | représente H ou $-CO-R^2$, |
| $X^1$ et $X^2$ | ne devant pas simultanément représenter chacun H, et |
| $R^1$ | représente H ou un reste aryle en $C_6$-$C_{14}$, éventuellement substitué par un groupe alkyle en $C_1$-$C_4$, par F, Cl, Br, I ou $NO_2$, |
| $R^2$ | représente un reste aryle en $C_6$-$C_{14}$, éventuellement substitué par un groupe alkyle en $C_1$-$C_4$, par F, Cl, Br, I ou $NO_2$, |
| Z | représente un groupe alkyle en $C_1$-$C_4$, aryle en $C_6$-$C_{10}$, alcoxy en $C_1$-$C_4$, aryloxy en $C_6$-$C_{10}$, dialkylamino en $C_1$-$C_4$, aryl ($C_6$-$C_{10}$)-alkyl ($C_1$-$C_4$)-amino, diarylamino en $C_6$-$C_{10}$, et |
| n | est un nombre entier valant 0 à 4. |

2. Procédé pour préparer des (poly)sulfone-cétones aromatiques, caractérisé en ce qu'on fait réagir des acides arylsulfiniques, ou leurs sels, avec des nitrobenzophénones de formule (III):

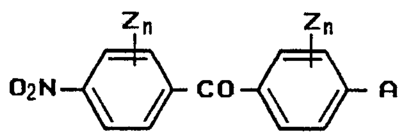

(III),

dans laquelle

| | |
|---|---|
| $Z_n$ | a le sens cité à la revendication 1, et |
| A | représente un atome d'hydrogène, un halogène ou un groupe nitro et, si A représente F, Cl, Br, I ou un groupe nitro, A peut éventuellement être mis en réaction avec l'acide arylsulfinique. |

3. Procédé pour préparer des sulfones (poly) aromatiques, contenant des groupes cétones ou aldéhydes, caractérisé en ce qu'on fait réagir les composés nitrés de formule (I) avec des acides disulfiniques aromatiques (ou avec leurs sels) répondant aux formules générales (IV) et (V):

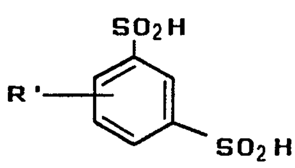

(IV),

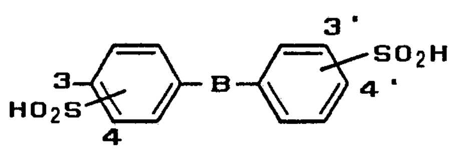

(V),

dans lesquelles

| | |
|---|---|
| R' | représente H, un groupe alkyle en $C_1$-$C_{18}$ ou un groupe aryle, |
| B | représente O ou $SO_2$, et |
| $X^1$ $X^2$ Z et | nont le sens indiqué dans le cas de la formule (I). |

4. Procédé pour préparer des sulfones aromatiques contenant des groupes cétones ou aldéhydes, caractérisé en ce qu'on fait réagir des composés nitrés de formule (I) avec des acides sulfiniques (ou avec leurs sels) de formule (II):

$Ar^1\ [SO_2\ M]_m$ (II)

dans laquelle

Ar[1] représente un groupe aryle en $C_6$-$C_{14}$ (m = 1), éventuellement substitué par un groupe alkyle en $C_1$-$C_4$ ou par de l'halogène, comme Cl, Br ou par $NO_2$, ou un groupe arylène en $C_6$-$C_{14}$ (m = 2),
m représente le nombre 1 ou 2, et
M représente un atome d'hydrogène ou de métal alcalin comme Li, Na,
K et le groupe $NR_4$, dans lequel R représente un groupe alkyle en $C_1$-$C_4$.

5. Procédé pour préparer des (poly)sulfones aromatiques selon la revendication 1, caractérisé en ce qu'on utilise le sel de lithium, de sodium, de potassium ou d'ammonium des acides sulfiniques ou leurs sels formés avec des bases organiques.

6. Procédé selon la revendication 1, caractérisé en ce qu'on conduit la réaction dans un solvant aprotique dipolaire, comme le diméthylsulfoxyde (DMSO) ou le diméthylformamide, ou bien l'on travaille dans des mélanges de solvants qui consistent surtout en des solvants aprotiques dipolaires.

7. Sulfone-cétones aromatiques de formule (VI):

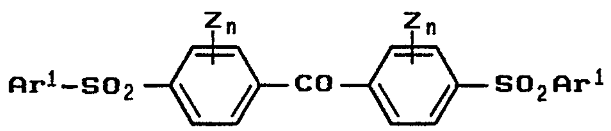

(VI),

dans laquelle

$Z_n$ a le sens indiqué dans le cas de la formule (I), et
Ar[1] représente un reste aromatique monofonctionnel en $C_6$-$C_{14}$, mais ne représente pas un groupe phényle, 3- ou 4-nitrophényle ou 3- ou 4-aminophényle.

8. Polysulfone-cétones de formule (VII):

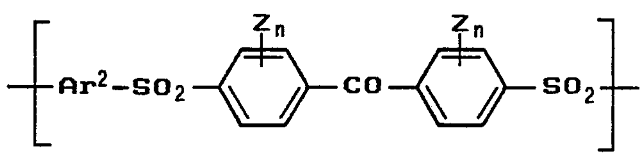

(VII),

dans laquelle

$Z_n$ a le sens indiqué dans le cas de ta formule (I), et
Ar[2] représente un reste aromatique difonctionnel en $C_6$-$C_{14}$.